# FASCICULE DE BREVET EUROPEEN

(11) **EP 0 861 329 B1**
(45) Date de publication et mention de la délivrance du brevet: **16.03.2005**
(21) Numéro de dépôt: 96938303.3
(22) Date de dépôt: 13.11.1996
(51) Int. Cl.: C12N 15/85, C07K 14/075, A61K 48/00

(54) **COMPLEXE PROTEIQUE DODECAEDRIQUE ADENOVIRAL, COMPOSITION LE CONTENANT ET SES APPLICATIONS**
ADENOVIRUS DODEKAHEDRISCHER PROTEINKOMPLEX, DIESER ENTHALTENDE ZUSAMMENSETZUNG UND VERWENDUNGEN DAVON
DODECAHEDRAL ADENOVIRAL PROTEIN COMPLEX, COMPOSITION CONTAINING SAME AND USES THEREOF

(30) Priorité: 13.11.1995 FR 9513406; 18.04.1996 FR 9604843
(43) Date de publication de la demande: 02.09.1998
(73) Titulaire: COMMISSARIAT A L'ENERGIE ATOMIQUE, 75015 Paris Cédex 15 (FR); CENTRE NATIONAL DE LA RECHERCHE SCIENTIFIQUE, 75016 Paris Cédex (FR)
(72) Inventeur: CHROBOCZEK, Jadwiga, F-38100 Grenoble (FR); FENDER, Pascal, F-38000 Grenoble (FR)
(74) Mandataire: Vialle-Presles, Marie José
(86) Numéro de dépôt international: PCT/FR1996/001790
(87) Numéro de publication internationale: WO 1997/018317

(56) Documents cités:
- WO-A-94/17832
- VIROLOGY, vol. 202, 1994, ORLANDO US, pages 782-795, XP002012045 LUCIE KARAYAN ET AL.: "Oligomerization of recombinant penton base of Adenovirus type 2 and its assembly with fiber in Baculovirus-infected cells"
- VIROLOGY, vol. 116, 30 Janvier 1982, ORLANDO US, pages 589-604, XP000565558 MARIE-LAURE BOUDIN ET AL. : "Assembly of Adenovirus penton base and fiber"

### LISTE DE SEQUENCES

(1) INFORMATIONS GENERALES:
   (i) DEPOSANT:
      (A) NOM: COMMISSARIAT A L'ENERGIE ATOMIQUE
      (B) RUE: 31-33 RUE DE LA FEDERATION
      (C) VILLE: PARIS
      (E) PAYS: FRANCE
      (F) CODE POSTAL: 75015

      (A) NOM: CENTRE NATIONAL DE LA RECHERCHE SCIENTIFIQUE-CNRS
      (B) RUE: 3 RUE MICHEL ANGE
      (C) VILLE: PARIS
      (E) PAYS: FRANCE
      (F) CODE POSTAL: 75794 CEDEX

      (A) NOM: CHROBOCZEK Jadwiga
      (B) RUE: 22 COURS DE LA LIBERATION
      (C) VILLE: GRENOBLE
      (E) PAYS: FRANCE
      (F) CODE POSTAL: 38100

      (A) NOM: FENDER PASCAL
      (B) RUE: 67 AVENUE ALSACE LORRAINE, APPT 607
      (C) VILLE: GRENOBLE
      (E) PAYS: FRANCE
      (F) CODE POSTAL: 38000
   (ii) TITRE DE L' INVENTION: COMPLEXE PROTEIQUE ADENOVIRAL, PROCEDE DE PREPARATION, COMPOSITION LE CONTENANT ET SES APPLICATIONS.
   (iii) NOMBRE DE SEQUENCES: 9
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
   (vi) DONNEES DE LA DEMANDE ANTERIEURE:
      (A) NUMERO DE LA DEMANDE: FR 95 13406
      (B) DATE DE DEPOT: 13-NOV-1995
   (vi) DONNEES DE LA DEMANDE ANTERIEURE:
      (A) NUMERO DE LA DEMANDE: FR 96 04843
      (B) DATE DE DEPOT: 18-APR-1996
(2) INFORMATIONS POUR LA SEQ ID NO: 1:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "amorce"
   (xi) DESCRIPTION DE LA SEQUENCE: SEQ ID NO: 1:
   GGATCCGATG AGGAGACGAG CC 22
(2) INFORMATIONS POUR LA SEQ ID NO: 2:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "amorce"
   (xi) DESCRIPTION DE LA SEQUENCE: SEQ ID NO: 2:
   GGATCCTTAG AAAGTGCGGC TTG 23
(2) INFORMATIONS POUR LA SEQ ID NO: 3:
   (i) CARACTERISTIQUES DE LA SEQUENCE:
      (A) LONGUEUR: 30 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "amorce"
   (xi) DESCRIPTION DE LA SEQUENCE: SEQ ID NO: 3:
   TTTCTTGAAT TCCAGATGGC CAAGCGAGCT                    30
(2) INFORMATIONS POUR LA SEQ ID NO: 4:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "amorce"
   (xi) DESCRIPTION DE LA SEQUENCE: SEQ ID NO: 4:
   AAAAGGAATT CCAATAAAAA ATGTTG                        26
(2) INFORMATIONS POUR LA SEQ ID NO: 5:
   (i) CARACTERISTIQUES DE LA SEQUENCE:
      (A) LONGUEUR: 40 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 5:
   Ala Lys Arg Ala Arg Leu Ser Thr Ser Phe Asn Pro Val Tyr Pro Tyr
   1               5                   10                  15
   Glu Asp Glu Ser Lys Lys Lys Lys Lys Lys Lys Lys Lys Lys Lys Lys
               20                  25                  30
   Lys Lys Lys Lys Lys Lys Lys Lys
           35                  40
(2) INFORMATIONS POUR LA SEQ ID NO: 6:
   (i) CARACTERISTIQUES DE LA SEQUENCE:
      (A) LONGUEUR: 50 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 6:
   Ala Lys Arg Ala Arg Leu Ser Thr Ser Phe Asn Pro Val Tyr Pro Tyr
   1               5                   10                  15
   Glu Asp Glu Ser Ser Arg Arg Arg Arg Arg Ser Arg Pro Thr Thr Val
               20                  25                  30
   Ser Asn Arg Leu Val Val Val Ser Thr Arg Arg Arg Ser Ser Arg Arg
           35                  40                  45
   Arg Arg
       50
(2) INFORMATIONS POUR LA SEQ ID NO: 7:
   (i) CARACTERISTIQUES DE LA SEQUENCE:
      (A) LONGUEUR: 42 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 7:
   Ser Phe Asn Pro Val Tyr Pro Tyr Glu Asp Glu Ser Ser Arg Arg Arg
   1               5                   10                  15
   Arg Arg Ser Arg Pro Thr Thr Val Ser Asn Arg Leu Val Val Val Ser
               20                  25                  30
   Thr Arg Arg Arg Ser Ser Arg Arg Arg Arg
           35                  40
(2) INFORMATIONS POUR LA SEQ ID NO: 8:
   (i) CARACTERISTIQUES DE LA SEQUENCE:
      (A) LONGUEUR: 26 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 8:
   Ser Phe Asn Pro Val Tyr Pro Tyr Glu Asp Glu Ser Met Arg Arg Ala
   1               5                   10                  15
   His His Arg Arg Arg Arg Ala Ser His Arg
               20                  25
(2) INFORMATIONS POUR LA SEQ ID NO: 9:
   (i) CARACTERISTIQUES DE LA SEQUENCE:
      (A) LONGUEUR: 25 acides aminés
      (B) TYPE: acide aminé
      (C) NOMBRE DE BRINS: simple
      (D) CONFIGURATION: linéaire
   (ii) TYPE DE MOLECULE: peptide

   (xi) DESCRIPTION DE LA SEQUENCE: SEQ ID NO: 9:
   Ser Phe Asn Pro Val Tyr Pro Tyr Glu Asp Glu Ser Gly Arg Arg Arg
   1               5                   10                  15
   Lys Arg Thr Ala Thr Arg Arg Arg Ser
               20                  25

## Description

La présente invention est relative à un complexe protéique adénoviral natif ou recombinant, à une composition pharmaceutique comprenant ledit complexe protéique ainsi qu'à leurs applications dans le traitement (médicaments) et la prévention (vaccins) des maladies humaines et animales. Ledit complexe protéique est notamment apte à dispenser à des cellules cibles convenables, des séquences nucléiques, des protéines, des peptides ou des substances chimiques d'intérêt.

La présente invention est également relative à des vecteurs d'expression dudit complexe protéique.

Les adénovirus constituent une famille de virus animaux à ADN regroupant plus de 90 sérotypes infectant différentes espèces, dont presque 50 sérotypes humains différents. Le tropisme cellulaire des adénovirus a pour conséquence l'infection des voies respiratoires, gastro-intestinales, urinaires et des yeux.

Ces virus sont responsables de 3 % de la totalité des infections chez l'homme, de 10 % des pneumonies infantiles et de 15 % des infections intestinales chez l'enfant.

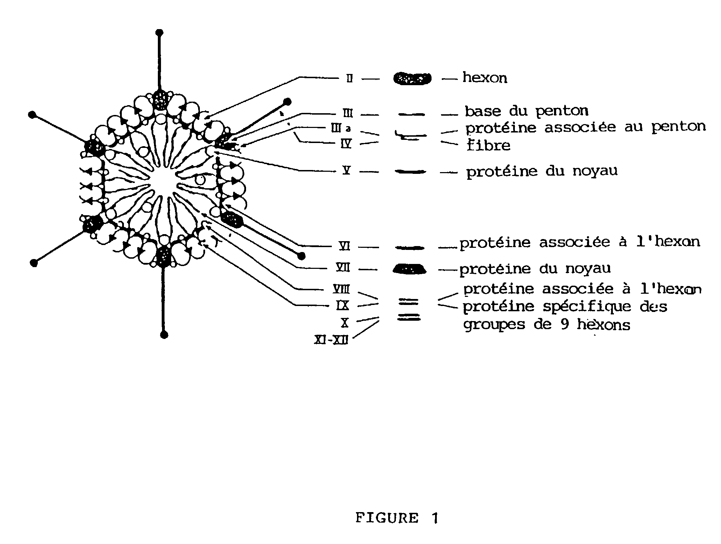

La particule d'adénovirus est relativement complexe et comprend plusieurs sous-structures ; en particulier, la partie externe ou capside est formée majoritairement de trois protéines : l'hexon, la base du penton et la fibre (voir figure 1).

Le penton, un complexe non covalent formé d'une protéine trimérique, la fibre et d'une protéine pentamérique, la base du penton, forme les sommets de l'icosaèdre viral.

Chacune des deux protéines formant le penton joue un rôle fondamental dans l'infection : la fibre permet l'attachement du virion à un récepteur cellulaire ; la base du penton permet l'internalisation du virion, probablement grâce à l'interaction avec les intégrines cellulaires (liaison aux intégrines, récepteurs de la vitronectine et de la fibronectine, par l'intermédiaire de la séquence arg-gly-asp présente au niveau de la base du penton) (Wickham et al., Cell, 1993, **73,** 309-319). Il existe en outre des observations qui suggèrent que la base du penton a une activité endosomolytique, permettant l'échappement dans le cytoplasme des molécules co-internalisées avec le virus (Seth et al., *Virus Attachment and Entry into Cells,* 1986, Ed. R.L. Crowell & K. Lonberg-Holm, 191-195).

La liaison des adénovirus aux cellules et leur internalisation sont donc deux événements distincts mais qui coopèrent.

La capacité à infecter des cellules quiescentes variées fait de l'adénovirus un vecteur de choix pour la thérapie génique.

Les méthodes couramment employées sont basées sur l'administration répétée d'adénovirus recombinants, déficients pour la réplication, véhiculant le gène-cible (Demandes internationales PCT WO 95/02697 et WO 95/14101, au nom de Rhone-Poulenc Rorer SA).

Toutefois, les traitements préconisés avec de tels adénovirus déficients présentent au moins les inconvénients suivants :
- risque de restauration en *trans* de la pathogénicité du virus recombinant chez un sujet traité et simultanément infecté par un adénovirus sauvage ;
- réactions immunitaires et inflammatoires, lors de l'administration répétée de l'adénovirus recombinant, dues à l'introduction massive de particules virales apportant des protéines étrangères,
qui mettent un frein à leur utilisation en tant que vecteur en thérapie génique, chez l'homme.

Pour éviter de tels inconvénients, il a été proposé d'utiliser uniquement le penton ou la base du penton, indépendamment du reste de génome de l'adénovirus, pour faciliter le transfert de gènes exogènes dans des cellules hôtes (Demande Internationale PCT WO 94/17832, au nom de The Scripps Research Institute). Bien qu'une telle approche présente des avantages par rapport aux adénovirus recombinants déficients pour la réplication, de telles structures (penton ou base du penton) sont fragiles, notamment la base du penton, qui peut être détruite par protéolyse.

En conséquence, le Demandeur s'est donné pour but de pourvoir à un vecteur utilisable en thérapie génique qui réponde mieux aux besoins de la pratique que les vecteurs de l'Art antérieur ; un tel vecteur ne présente ni les inconvénients des adénovirus recombinants, ni la fragilité du penton ou de la base du penton, telle que précisée ci-dessus.

La présente invention a pour objet un complexe protéique adénoviral, caractérisé en ce qu'il est constitué soit :
- de 12 pentons, comprenant chacun au moins une fibre et une base de penton, à l'exclusion de tout autre élément constitutif du génome d'un adénovirus, lesquelles fibre(s) et base du penton sont dérivées soit du même adénovirus, soit d'adénovirus différents, lesdits pentons étant liés par les bases de penton et formant une structure en dodécaèdre, stable aux enzymes protéolytiques, lequel complexe présente un poids moléculaire compris entre 4,8.10⁶ et 6,6.10⁶ ; de tels complexes sont dénommés ci-après complexe dodécaèdre-fibre ou dodécaèdre-penton ;
- soit de 12 bases de penton, à l'exclusion de tout autre élément constitutif du génome d'un adénovirus, lesquelles bases de penton sont dérivées soit du même adénovirus, soit d'adénovirus différents, et forment une structure en dodécaèdre, stable aux enzymes protéolytiques et en ce qu'il présente un poids moléculaire compris entre 3,2.10⁶ et 4.10⁶ ; de tels complexes sont dénommés ci-après complexes dodécaèdre-base.

Lesdits complexes ne comprennent donc aucun élément du génome d'un desdits adénovirus. Lesdits complexes protéiques dodécaèdriques natifs ou recombinants, facilitent le transfert de gènes étrangers, de séquences nucléiques, de protéines, de peptides ou de molécules chimiques dans les cellules cibles.

Conformément à l'invention, lesdits adénovirus sont sélectionnés parmi les adénovirus humains et notamment l'adénovirus de type 2 (Ad2), l'adénovirus de type 3 (Ad3), l'adénovirus de type 5 (Ad5), l'adénovirus de type 4 (Ad4), l'adénovirus de type 7 (Ad7), l'adénovirus de type 9 (Ad9), l'adénovirus de type 11 (Ad11), l'adénovirus de type 15 (Ad15) ou les adénovirus entériques (Ad40 et Ad41) et les adénovirus aviaires.

Selon un mode de réalisation avantageux desdits complexes protéiques dodécaèdriques recombinants, au moins l'un de ses constituants (base et/ou fibre) peut être modifié pour augmenter l'affinité vis-à-vis d'un type cellulaire particulier.

Un tel complexe protéique ainsi modifié ne présente pas de diminution dans ses propriétés d'attachement à la cellule cible et d'internalisation dans ladite cellule, d'une part par rapport à l'adénovirus sauvage et d'autre part par rapport au complexe selon l'invention dans lequel lesdits constituants ne sont pas modifiés.

Par exemple, une séquence d'attachement à d'autres récepteurs, tels que le récepteur CD4 présent sur les cellules T, peut être incorporée sur la fibre par des techniques d'ADN recombinant (association ligand-fibre).

On peut citer comme exemples de ligands appropriés, la boucle V3 de la gp120 d'HIV (liaison avec le CD4), la transferrine (liaison au récepteur de la transferrine), les LDL (liaison aux récepteurs LDL), des protéines déglycosylées et des anticorps.

Un complexe protéique dodécaèdrique préféré selon l'invention est constitué soit de 12 bases de penton, correspondant à celles d'un adénovirus sélectionné dans le groupe constitué par Ad3, Ad4, Ad7, Ad9, Ad11 et Ad15, soit de 12 pentons dont les séquences des fibres correspondent à celles de l'un quelconque des adénovirus humains, entériques ou aviaires précités et dont les séquences des bases du penton correspondent à celles d'un adénovirus sélectionné dans le groupe constitué par Ad3, Ad4, Ad7, Ad9, Ad11 et Ad15, de préférence à celles de l'adénovirus de type 3 (Ad3).

Un autre complexe protéique dodécaèdrique préféré selon l'invention est constitué de 12 pentons, dont les séquences des fibres correspondent à celles de l'adénovirus de type 2 (Ad2) ou de l'adénovirus de type 5 (Ad5) et dont les séquences des bases du penton correspondent à celles de l'adénovirus de type 3 (Ad3).

L'utilisation du complexe protéique adénoviral selon l'invention à la place du virion de l'adénovirus élimine le danger de l'infection fortuite par l'adénovirus et est accompagnée par de beaucoup plus faibles réactions immunitaires et inflammatoires ; il est en outre particulièrement stable et la présence des 12 bases du penton augmente de manière significative la vitesse de lyse des endosomes (passage cytoplasmique plus rapide), par rapport à une structure ne contenant qu'un penton ou qu'une base.

Selon un autre mode de réalisation avantageux dudit complexe protéique adénoviral recombinant, il comprend en outre au moins un hexon ou une autre protéine virale.

La présente invention a également pour objet une composition pharmaceutique, caractérisée en ce qu'elle comprend essentiellement un complexe protéique adénoviral selon l'invention et au moins une autre substance chimique.

Selon un mode de réalisation avantageux de ladite composition, ladite autre substance chimique est sélectionnée dans le groupe constitué par des séquences nucléiques, des protéines, des peptides et des substances chimiques pharmacologiquement actives.

Lorsque la substance chimique est une protéine, cette dernière a de préférence des propriétés immunoprotectrices et antigéniques, et est particulièrement bien adaptée à la préparation d'un vaccin.

Lorsque la substance chimique est une substance chimique pharmacologiquement active, elle est notamment sélectionnée parmi les toxines cellulaires, telles que les agents anticancéreux et notamment les anthracyclines.

Dans ce dernier cas, la composition selon l'invention a l'avantage d'éviter le mécanisme de résistance habituellement rencontré avec les anticancéreux. En effet, le développement d'une résistance à une chimiothérapie antitumorale constitue un obstacle majeur au traitement des cancers humains. Cette résistance est due en grande partie à l'induction de l'activité d'une pompe extracellulaire très efficace qui ne permet pas le transport de la substance pharmacologiquement active à l'intérieur de la cellule. Or, de manière surprenante, la composition selon l'invention permet l'introduction de la substance pharmacologiquement active dans la cellule, en contournant ce mécanisme de résistance.

Lorsque la substance active est une séquence nucléique, elle est sélectionnée parmi les gènes qui codent pour un polypeptide présentant une activité thérapeutique, les séquences anti-sens et les ribozymes.

Dans le cas d'une séquence codante, elle comprend en outre un promoteur actif pour l'expression du polypeptide.

Selon une disposition avantageuse de ce mode de réalisation, le promoteur est sélectionné dans le groupe constitué par des promoteurs constitutifs et des promoteurs inductibles.

Ladite substance chimique active peut être liée au complexe protéique adénoviral selon l'invention au moins de deux façons différentes :
- soit par l'intermédiaire d'une liaison passagère, telle qu'une liaison ionique ou hydrophobe, qui peut être détruite et permet ainsi le passage de la substance active dans le noyau (expression d'un gène au niveau du noyau) (ligand sélectionné parmi les substances aptes à produire une liaison ionique ou hydrophobe avec ladite substance active) ;
- soit par l'intermédiaire d'une liaison stable, telle qu'une liaison covalente, par exemple, qui retient la substance active dans le cytoplasme ; un tel type de liaison est en particulier intéressant pour la préparation de composition vaccinales (persistance de la substance active dans le cytoplasme) (ligand sélectionné parmi les substances aptes à produire une liaison covalente avec ladite substance active).

En effet, ladite substance chimique, notamment une séquence d'acide nucléique, une protéine ou un peptide, peut être associée (de manière covalente ou non-covalente) à un ligand convenable.

Parmi les ligands convenables, on peut citer les peptides dont la partie N-terminale comprend la séquence en aminoacides N-terminale d'une fibre d'adénovirus de n'importe quel sérotype (zone d'attachement au complexe protéique selon l'invention) et dont la partie C-terminale comprend :
- une polylysine (séquence qui permet l'attachement de la substance active, notamment lorsqu'il s'agit d'acide nucléique),
- une polyarginine,
- une partie ou la séquence complète d'une protéine de coeur de n'importe quel adénovirus (protéine VII, protéine µ des adénovirus humains ou protéines homologues rencontrées dans les adénovirus présents dans d'autres animaux),
- une cystéine, ou
- un complexe transferrine/poly-L-lysine, qui s'associe à ladite séquence nucléique ou à ladite protéine, pour former un conjugué.

De tels peptides, dénommés peptides bifonctionnels, sont liés par leur partie N-terminale au complexe protéique adénoviral selon l'invention et par leur partie C-terminale, à un ADN plasmidique à transférer.

Par exemple, le peptide comprenant une polylysine à son extrémité C-terminale permet une liaison de type ionique, un peptide comprenant une cystéine à son extrémité C-terminale permet une liaison de type covalent et l'association d'un conjugué acide nucléique ou protéine et transferrine/poly-L-Lysine, dans lequel la transferrine se lie aux récepteurs de la transferrine à la surface des cellules cibles, avec le complexe protéique selon l'invention permet un meilleur transport de la substance chimique, des endosomes vers le cytoplasme.

Un autre mode de transfert des séquences nucléiques ou des protéines est possible, en utilisant une réaction de complexation avidine-biotine et un anticorps anti-fibre, anti-base du penton ou anti-complexe protéique dodécaèdrique. Une telle composition met en oeuvre : un complexe protéique dodécaèdrique selon l'invention, un anticorps qui réagit soit avec la fibre, soit avec la base du penton, soit avec le complexe protéique dodécaèdrique, mais n'inhibe pas la fonctionnalité dudit complexe protéique, un vecteur contenant une séquence nucléique d'intérêt dans laquelle au moins un nucléotide est biotinylé, éventuellement liée à une séquence marqueur ou une protéine biotinylée et une protéine chimérique consistant en protéine A liée à de la streptavidine (SA-PA). Du fait qu'un nucléotide ou la protéine est biotinylé, ils peuvent se lier à la protéine chimérique SA-PA. Dans une telle composition, l'ADN biotinylé ou la protéine biotinylée se lie à la protéine chimérique SA-PA ; le complexe ADN lié à la SA-PA se lie ensuite via la moitié PA, à un anticorps qui réagit soit avec une fibre, soit avec une base du penton, soit avec le complexe protéique dodécaèdrique. Le produit complexe ainsi formé comprenant PA-anticorps immobilisé-complexe protéique dodécaèdrique se lie, par l'intermédiaire du complexe protéique, aux récepteurs spécifiques de ce dernier, exprimés à la surface des cellules cibles.

Dans tous les cas, la séquence d'acide nucléique exogène, la protéine d'intérêt ou toute autre substance chimique, incluse dans ou associée à la composition selon l'invention, pénètre dans la cellule (internalisation) par l'intermédiaire du complexe protéique dodécaèdrique selon l'invention.

De manière surprenante, l'interaction dodécaèdre-récepteur cellulaire accroît, de manière significative, à la fois l'intemalisation de la composition selon l'invention et la perméabilité des endosomes, ce qui augmente, de manière significative, le passage de l'acide nucléique exogène, de la protéine d'intérêt ou de toute autre substance chimique, des endosomes vers le cytoplasme, en comparaison avec l'utilisation d'une composition ne contenant qu'un seul penton.

Selon un autre mode de réalisation de ladite composition pharmaceutique, elle comprend, en outre, au moins un véhicule pharmaceutiquement acceptable.

Parmi les véhicules pharmaceutiquement acceptables, on peut citer aussi bien des véhicules adaptés à la voie d'administration recherchée tels que l'eau, des sels, le dextrose, le glycérol, l'éthanol des huiles végétales, le propylène glycol, le polyéthylène glycol, l'alcool benzylique (administration parentérale ou préparations liquides), que des liposomes ou d'autres polymères (par exemple des polymères cationiques).

De telles compositions peuvent, en outre, contenir des agents mouillants ou émulsifiants, des agents isotoniques, des agents de dissolution, des stabilisants, des colorants, des agents antiseptiques etc...

Conformément à l'invention, ladite substance chimique active est soit incluse dans ledit complexe protéique dodécaèdrique selon l'invention, soit associée audit complexe ; dans les deux cas, elle peut être libre ou liée audit complexe.

Les compositions selon l'invention peuvent être administrées sous différentes formes et par différentes voies, telles que voie pulmonaire (aérosol), voie intrapéritonéale, voie parentérale ou implant chirurgical.

Les compositions selon l'invention ont de nombreuses applications comme médicaments, en médecine humaine et vétérinaire :
- en thérapie génique humaine et animale, notamment dans les maladies héréditaires impliquant l'épithélium respiratoire telles que l'emphysème ou la mucoviscidose,
- comme agents antiviraux (séquences anti-sens ou ribozymes),
- comme agents immunogènes ou vaccinaux,
- comme agents anti-bactériens, anti-cancéreux etc...

La présente invention a, également, pour objet une composition comprenant essentiellement un complexe protéique dodécaèdrique adénoviral, tel que défini ci-dessus et une substance chimique sélectionnée parmi les séquences nucléotidiques, les protéines et les substance chimiques actives pharmacologiquement, en tant que médicament ou en tant que vaccin.

La présente invention a également pour objet une composition, comprenant un complexe protéique dodécaèdrique adénoviral, tel que défini ci-dessus et une substance chimique sélectionnée parmi les séquences nucléotidiques, les substances immunogènes, et les substances anticancéreuses, pour son utilisation dans le traitement des maladies humaines ou animales impliquant des cellules exprimant des récepteurs spécifiques des fibres d'adénovirus et/ou des cellules exprimant les récepteurs des intégrines, notamment des intégrines αᵥβ₃ ou αᵥβ₅, telles que les cellules épithéliales, les cellules endothéliales, les plaquettes sanguines, les cellules lymphoïdes et les cellules cancéreuses et libérant une quantité efficace de ladite substance chimique active aux dites cellules.

La présente invention a, en outre, pour objet un procédé de préparation dudit complexe protéique dodécaèdrique adénoviral conforme à l'invention.

Un tel procédé comprend, de préférence les étapes suivantes :
(1) le clonage séparé ou simultané du (ou des) gène(s) codant pour la/les fibre(s) d'un adénovirus, le gène codant pour la base du penton d'un adénovirus et éventuellement le gène codant pour l'hexon d'un adénovirus, lesdits gènes étant issus soit du même adénovirus, soit d'adénovirus différents, dans au moins un vecteur baculovirus, pour l'obtention de plusieurs plasmides recombinants contenant soit un gène, soit deux gènes (expression simultanée des deux fibres dans le cas d'un adénovirus contenant deux fibres par penton) ou pour l'obtention d'un plasmide recombinant contenant simultanément deux gènes (ou trois gènes, dans le cas de l'utilisation d'un adénovirus contenant deux fibres par penton) ; dans ce dernier cas, le vecteur baculovirus utilisé comprend une cassette de double ou de triple expression, alors que dans le cas où le complexe protéique selon l'invention comprend en outre au moins un hexon, ledit vecteur baculovirus peut comprendre une cassette d'expression multiple ;
(2) la co-transfection du ou des plasmides recombinants et d'un fragment linéarisé d'ADN de baculovirus, dans une cellule d'insecte ;
(3) le criblage et la sélection des clones recombinants de baculovirus exprimant, séparément ou à la fois la/les fibres, la base du penton et éventuellement l'hexon ;
(4) la purification des clones sélectionnés correspondant à des baculovirus recombinants replicables, contenant un ou plusieurs desdits gènes, issus d'au moins un adénovirus et exprimant les protéines correspondantes ;
(5) l'extraction des protéines exprimées par lesdits baculovirus recombinants, comprenant essentiellement la lyse des cellules d'insecte, l'élimination des débris cellulaires et des noyaux par centrifugation et la récupération du surnageant ; et
(6) la purification du complexe protéique dodécaèdrique selon l'invention, par application dudit surnageant (extrait de protéines) sur un gradient de saccharose, dont la fourchette de concentrations varie en fonction du poids moléculaire et de la densité des dodécaèdres obtenus et récupération des fractions, dans la zone des concentrations élevées en saccharose ; par exemple, pour les complexes formés à partir d'Ad2 et/ou d'Ad3, le gradient de saccharose varie de 15 à 40 %, et l'on récupère la fraction correspondant à une concentration en saccharose comprise entre 31 et 38 %.

Selon un mode de mise en oeuvre avantageux dudit procédé, les cellules d'insecte de l'étape (2) sont sélectionnées dans le groupe constitué par les cellules de *Spodoptera frugiperda* et les cellules de *Trichoplusia ni.*

En variante, la co-transfection de l'étape (2) est mise en oeuvre dans des cellules de *Spodoptera frugiperda* et le procédé comprend une deuxième étape (4') de transfection des clones purifiés de baculovirus recombinants obtenus à l'étape (4), dans des cellules d'insecte *Trichoplusia ni.*

Selon un autre mode de mise en oeuvre avantageux dudit procédé, le vecteur baculovirus comprenant une cassette de double, triple ou multiple expression selon l'étape (1) est un vecteur comprenant deux ou trois promoteurs forts.

On peut citer, par exemple, le vecteur de transfert pAcUW31 (Clontech®), qui comprend (i) le promoteur de la polyédrine et le promoteur p10, le promoteur de la polyédrine étant suivi d'un site unique de clonage BamHI et des signaux de polyadénylation de la polyédrine, alors que le promoteur p10 est suivi des sites uniques de clonage Bgl II et EcoRI et d'un signal de polyadénylation du SV40, (ii) une origine de réplication M13, (iii) une origine de réplication pUC et (iv) un gène reporter, tel que luciférase, β-galactosidase ou gène de résistance à un antibiotique (ampicilline, par exemple).

Selon un autre mode de mise en oeuvre avantageux dudit procédé, ledit fragment linéarisé d'ADN de baculovirus de l'étape (2) est obtenu à partir d'un vecteur contenant trois sites de restriction Bsu36I et le gène lacZ à la place de la séquence codant pour la polyédrine, par exemple, le vecteur d'ADN viral BacPAK6® (Clontech), lequel vecteur est digéré par l'enzyme de restriction Bsu36I avant ladite co-transfection de l'étape (2).

Selon un autre mode de mise en oeuvre avantageux dudit procédé, la cellule d'insecte *Spodoptera frugiperda* de l'étape (2) est sélectionnée dans le groupe constitué par les cellules Sf21 et les cellules Sf9.

Selon un autre mode de mise en oeuvre avantageux dudit procédé, la purification des clones de l'étape (4) est réalisée par la mise en oeuvre d'au moins deux sous-clonages successifs.

Selon un autre mode de mise en oeuvre avantageux dudit procédé, les cellules d'insecte *Trichoplusia ni* de l'étape (5) sont des cellules BTI-TN-5B1-4 (*High Five*® d'Invitrogen).

En variante, la transfection de l'étape (4') comprend la co-transfection de baculovirus recombinants qui contiennent les deux gènes (ou les trois gènes, dans le cas d'un adénovirus contenant deux fibres par penton), issus d'adénovirus précités, éventuellement associés à des baculovirus recombinants qui contiennent un seul des deux gènes issus d'adénovirus.

Conformément à l'invention, la transfection peut être réalisée par différentes méthodes, selon le vecteur utilisé : méthode au phosphate de calcium, méthode au DEAE-dextran, méthode de transfert stable, électroporation, méthode aux liposomes.

Selon un autre mode de mise en oeuvre avantageux dudit procédé, l'étape (5) d'extraction de l'ensemble des protéines exprimées par le baculovirus recombinant sélectionné comprend l'obtention d'un extrait cellulaire par lyse des cellules de *Trichoplusia ni,* au moyen de plusieurs cycles de congélation-décongélation, dans un tampon Tris 10 mM pH8, l'élimination des débris cellulaires et des noyaux présents dans ledit extrait cellulaire par centrifugation pendant quelques minutes à 7 000-10 000 g et la récupération du surnageant.

Selon encore un autre mode de mise en oeuvre avantageux dudit procédé, l'étape (6) de purification du complexe protéique selon l'invention comprend une centrifugation à 121 000 g au haut du tube et à 275 000 g au fond du tube, pendant 17 à 19 heures et à froid (4°C).

Selon encore un autre mode de mise en oeuvre avantageux du procédé, le complexe protéique dodécaèdrique purifiée à l'étape (6) est soumis à une concentration.

En variante, l'étape (2) comprend l'isolement de l'ADN recombinant (plasmide recombinant) obtenu dans une bactérie et la transfection de cellules d'insectes avec ledit ADN recombinant, conformément au procédé GIBCO-BRL, dénommé Bac-to-Bac® *Baculovirus expression system.*

Les dodécaèdres natifs sont avantageusement obtenus par extraction des protéines exprimées par des cellules infectées par un adénovirus (voir étape (5) ci-dessus) et purification du complexe dodécaèdrique sur un gradient de saccharose, dans les mêmes conditions que celles énoncées ci-dessus, pour la préparation des dodécaèdres recombinants.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la figure 1 représente une vue schématique d'un adénovirus ;
- les figures 2A et 2B montrent les résultats obtenus à partir de cellules exprimant la fibre et la base du penton, issues d'un Ad3 (dodécaèdre Ad3) et représentent deux gels d'électrophorèse SDS-PAGE, obtenus à partir des fractions du gradient de saccharose : figure 2A : résultat du transfert de protéine (*Western blot ou immuno blot*) ; figure 2B : résultat après coloration au Bleu de Coomassie ;
- les figures 3 et 4 illustrent le rôle de la base du penton dans la formation du dodécaèdre ;
- les figures 5 et 6 représentent des micrographies électroniques respectivement de dodécaèdre-fibre et de dodécaèdre-base d'Ad3, colorés négativement avec du silicotungstate de sodium à 1 % ; ces micrographies sont réalisées à partir d'un échantillon de matériel obtenu après centrifugation du gradient de saccharose variant de 31 à 38 %, mélangé, dialysé et concentré ;
- la figure 7 représente un dodécaèdre chimérique ;
- les figures 8 et 9 illustrent l'internalisation du complexe protéique dodécaèdrique selon l'invention, dans des cellules HeLa ;
- la figure 10 illustre l'internalisation d'un dodécaèdre-base et d'un dodécaèdre-fibre : les histogrammes □ illustrent l'internalisation du dodécaèdre-fibre et les histogrammes ■ illustrent l'internalisation d'un dodécaèdre-base ; cette figure comprend, en abscisse, la quantité de particules virales/cellule (=MOI ou *Multiplicity of Infection*) et en ordonnée, les unités de fluorescence (unités de fluorescence arbitraires) ;
- la figure 11 illustre la transfection d'un plasmide contenant le gène codant pour la luciférase en présence d'un dodécaèdre-penton ou d'un dodécaèdre-base, comparée avec la transfection effectuée par l'adénovirus recombinant apportant le même gène ; elle comporte, en abscisse, la quantité de particules virales/cellule (=MOI ou *Multiplicity of Infection*) et en ordonnée, la quantité de lumière relative (=RLU ou Relative Light Unit) ;
- les figures 12A et 12B représentent des micrographies électroniques respectivement de complexes ADN/peptide/dodécaèdre-penton (A) et de complexes ADN/peptide/dodécaèdre-base (B), colorés négativement avec du silicotungstate de sodium à 1 % ;
- la figure 13 montre l'interaction du peptide bifonctionnel avec un complexe protéique dodécaédrique selon l'invention et un ADN plasmidique ; cette figure représente un gel d'électrophorèse.

### EXEMPLE 1 : Clonage, expression et purification du dodécaèdre d'adénovirus de type 3.

### 1. Amplification par PCR des gènes de fibres et de la base du penton.

En utilisant la PCR, il est possible de synthétiser des séquences nucléotidiques codant pour des polypeptides utiles, qui peuvent être insérés dans un vecteur approprié et utilisés pour transformer une cellule spécifique et les exprimer dans cette dernière.

La méthode pour produire les gènes exprimant la base du penton et la fibre du dodécaèdre d'adénovirus selon l'invention dépend de la présélection d'oligonucléotides en tant qu'amorces dans une réaction de polymérisation en chaîne (PCR).

La séquence des gènes de la base du penton et de la fibre de l'Ad3 a été décrite respectivement dans CUZANGE et al., Gene, 1994, **146**, 257-259 et SIGNAS et al., J. Virol., 1985, **53**, 672-678.

Les amorces en 5' et en 3' utilisées pour amplifier le gène codant pour la base du penton sont respectivement 5'-GGATCCG**ATG**AGGAGACGAGCC-3' (SEQ ID N°1) et 5'-GGATCC*TTA*GAAAGTGCGGCTTG-3' (SEQ ID N°2).

Les amorces 5' et 3' utilisées pour amplifier le gène codant pour la fibre sont respectivement 5'-TTTCTTGAATTCCAG**ATG**GCCAAGCGAGCT-3' (SEQ ID N°3) et 5'-AAAAGGAATTCCAATAAAAAATGTTG-3' (SEQ ID N°4).

Les sites de clonage sont soulignés et sont respectivement BamH I, BamH I, EcoR I, EcoR I. Le codon d'initiation ATG est en gras et le codon stop TAA (ou TTA dans le brin complémentaire) est en italique.

Les séquences des fibres et bases du penton de l'Ad2 et de l'Ad5 sont notamment décrites dans Chroboczek J. et al., Virology, 1987, **161**, 549-554 et Virology, 1992, 186, 280-285 ; Neumann R. et al., Gene, 1988 69, 153-157.

L'amplification par PCR est réalisée sur les extraits d'ADN isolés à partir du virus, obtenus par propagation dans des cellules HeLa comme décrit dans HORWITZ (« *Adenoviridae and their replication »,* in *Virology,* Fields and Knipe, éds. Raven Press, New York, 1990).

Le tampon de PCR contient par exemple: KCl 50 mM, Tris-HCl à pH 8,3 10 mM, MgCl₂ 1,5 mM, gélatine à 0,001%, ATP 200 µM, dTTP 200 µM, dCTP 200 µM, dGTP 200 µM et 2,5 unités d'ADN polymérase de *Thermus aquaticus*/100 µl de tampon. La PCR est réalisée dans les conditions suivantes : après dénaturation 2 minutes à 94°C, la PCR comprend 25 cycles de 1 min à 94°C, 1 min à 55°C et 1 min à 72°C.

### 2. Clonage des produits PCR dans le vecteur intermédiaire pCR-Script™.

Le vecteur de clonage pCR-Script™ (Stratagene, Catalogue n° 211190, 1994) présente une grande efficacité de ligation d'ADN, lors du clonage des produits PCR.

Les produits de l'amplification PCR sont fractionnés par électrophorèse sur gel d'agarose à 1% dans un tampon TAE ; les bandes convenables sont séparées et l'ADN est extrait selon la procédure GENECLEAN II® (Bio101, Inc.). Les fragments d'ADN contenant les gènes codant pour la base du penton et la fibre sont clonés, de manière séparée, dans le vecteur pCR-Script™ précité selon les indications du fournisseur.

Les clones sont ensuite traités pour obtenir l'ADN, selon le procédé décrit dans Sambrook et al (*Molecular Cloning, a Laboratory Manual,* deuxième édition), au chapitre concernant les minipréparations d'ADN plasmidique. Dans ces conditions, la présence des gènes codant pour la fibre et la base du penton de l'adénovirus est confirmée, en comparant la taille des fragments obtenus après digestion par BamHI et EcoRI avec des produits standards, sur gel d'agarose.

Les clones positifs sont amplifiés, l'ADN plasmidique est isolé et les gènes de la fibre et de la base du penton sont obtenus après digestion avec les mêmes enzymes de restriction (BamH I et EcoR I), conformément aux méthodes de génie génétique usuellement utilisées dans le clonage moléculaire (Sambrook et al, précité).

### 3. Clonage des gènes de fibres et de base du penton dans un vecteur d'expression.

Le vecteur de double-expression pAcUW31® (Clontech) est utilisé pour exprimer le dodécaèdre.

Premièrement, le gène de la base du penton est introduit au niveau du site BamHI de ce vecteur, en amont du promoteur de la polyédrine. Le clonage est vérifié par hybridation avec une sonde non-radioactive correspondant à la base du penton contenant du dUTP lié à de la fluorescéine conformément à la procédure du kit ECL® . L'orientation de l'insert par rapport au promoteur est déterminée dans les clones positifs par une analyse de restriction.

Dans une seconde étape, le gène codant pour la fibre est inséré au niveau du site EcoRI dudit vecteur, en aval du promoteur du gène p10. Le clonage et l'orientation de l'insert sont vérifiés comme précisé ci-dessus.

La séquence du gène codant pour la base et du gène codant pour la fibre sont vérifiées dans le plasmide résultant. Aucune mutation n'est détectée. De manière similaire, les vecteurs qui portent seulement soit le gène codant pour la fibre, soit le gène codant pour la base du penton sont préparés et analysés comme décrit ci-dessus.

Les plasmides d'expression qui portent soit les deux gènes, soit l'un des deux gènes d'intérêt, sont transformés dans une souche d'*E*. *coli* TG-1, comme décrit dans HANAHAN (J. Mol. Biol., 1983, **166,** 557-580) et une purification du plasmide à grande échelle est réalisée en utilisant les techniques de génie génétique bien connues (Sambrook et al,, précité).

On peut également, si nécessaire (dans le cas de l'utilisation d'adénovirus comprenant deux fibres), mettre en oeuvre un vecteur de triple expression, tel que décrit dans A.J. Belyaev et T. Roy (N.A.R., 1993, 21, 1219-1223).

### 4. Transfection dans des cellules d'insectes.

Les plasmides recombinants (100 ng) sont (séparément) cotransfectés avec un baculovirus linéarisé (Clontech : ADN viral BacPAK6® digéré par l'enzyme Bsu36I) en présence de lipofectine® (GIBCO) dans des cellules de *Spodoptera frugiperda* (Sf21) et des cellules de *Trichoplusia ni* (High-Five® ) selon la technique décrite par KITTS et al. (N.A.R., 1990, **18,** 19, 5667-5672). Cette méthode permet d'obtenir une efficacité de transformation supérieure à 80 %. Quelques clones sont choisis dans chaque cas et l'expression est détectée par la technique en *Western blot,* réalisée avec des anticorps de lapin polyclonaux spécifiques des antigènes de fibres et de bases du penton.

### 5. Purification des clones positifs.

Les isolats de baculovirus recombinants sont soumis à 3 repiquages sur plaques, suivis d'un titrage selon la méthode de KING et POSSEE (*The baculovirus expression system, A laboratory guide,* L.A. KING and R.D. POSSEE, ed.: CHAPMAN & HALL, 1992). L'expression des deux protéines est suivie par la technique en Western blot telle que précisée ci-dessus.

### 6. Purification de la protéine.

La cinétique de l'expression de dodécaèdre est suivie dans les deux types de cellules pendant 5 jours, sur les cellules adhérentes, cultivées à 27°C sur milieu TC 100 GIBCO contenant 5 % de sérum de veau fétal (cellules Sf21 ou Sf9) ou sur milieu TC 100 GIBCO contenant 10 % de sérum de veau fétal (cellules High Five® ). Dans la mesure où le rendement en expression protéique est significativement plus important dans les cellules High-Five® , une expression de protéines à grande échelle est réalisée uniquement dans ces cellules.

Les essais pour augmenter le taux d'expression par co-transfection dans des cellules d'insectes avec un baculovirus portant les deux gènes et différentes quantités de baculovirus ne portant qu'un seul gène, n'ont pas été significatifs.

3 jours après l'infection avec le baculovirus recombinant (multiplicité de l'infection 5), des cellules High-Five® sont récoltées dans un tampon Tris 10 mM pH 8, contenant des inhibiteurs de la protéase.

La lyse cellulaire est réalisée en mettant en oeuvre 3 cycles de congélation-décongélation dans le même tampon Tris. L'extrait de la lyse est débarrassé des éléments solides (débris cellulaires et noyaux) par centrifugation pendant 5 min à 7 000-10 000 g, puis est soumis à un gradient de saccharose variant de 15 à 40 % (11 ml) dans un tampon de gradient comprenant du glycérol 10 %, NaCl 150 mM, Tris-HCl 10 mM pH 7,4 et EDTA 2 mM.

Après 18 heures de centrifugation à 4°C dans un rotor Beckman SW 41, à 121 000 g, en haut du tube et à 275 000g en bas du tube ; l'échantillon ainsi traité est récupéré par le haut, par fractions de 800 µl.

Dans ces conditions, le dodécaèdre est récupéré dans les fractions contenant 31-38 % de saccharose. Ces fractions sont mélangées et dialysées contre un tampon de gradient sans saccharose et glycérol.

Le dodécaèdre est concentré par centrifugation sur Centritrep® (Amicon).

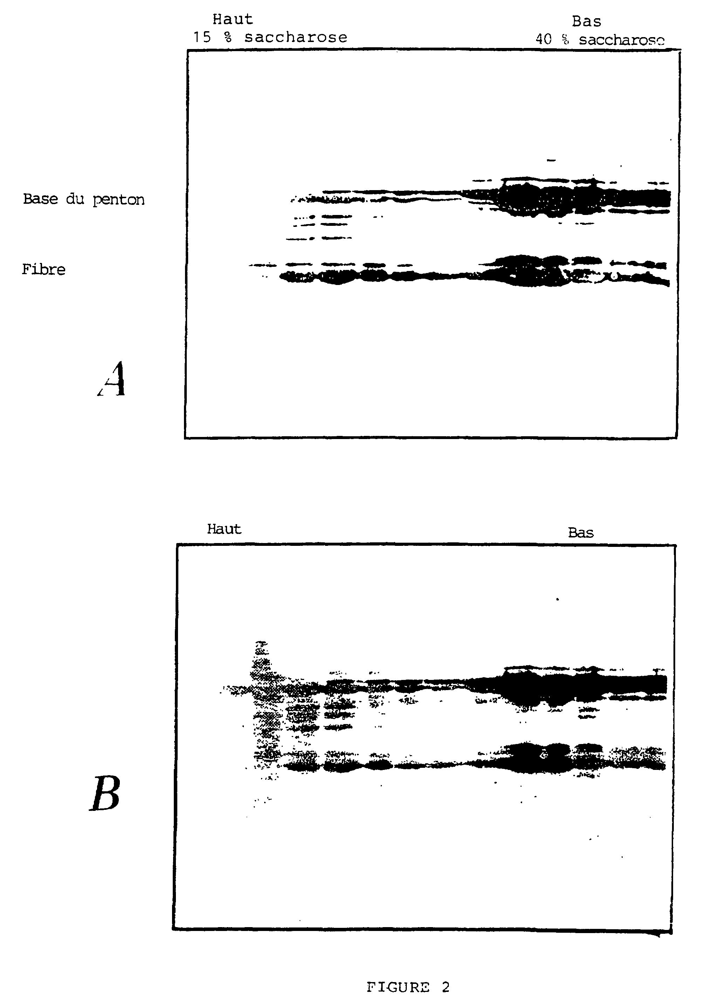

Les figures 2A et 2B illustrent les résultats obtenus après purification du dodécaèdre : elles sont obtenues à partir d'aliquots de chaque fraction, obtenue à partir du gradient de saccharose comme décrit ci-dessus, dénaturés à la chaleur en présence de SDS à 1 % et électrophorèse dans deux gels SDS-polyacrylamide à 10 % ; les protéines sont analysées sur l'un des gels par transfert de protéines, suivi d'une réaction avec des anticorps spécifiques anti-fibre et anti-base du penton (*Western blot*) (figure 2A) et sur le deuxième gel, par coloration au Bleu de Coomassie Brilliant. Les fractions situées en haut du tube contiennent les bases du pentons et fibres libres et les fractions récupérées dans la zone 31-38 % de saccharose, contiennent le complexe protéique dodécaèdrique selon l'invention.

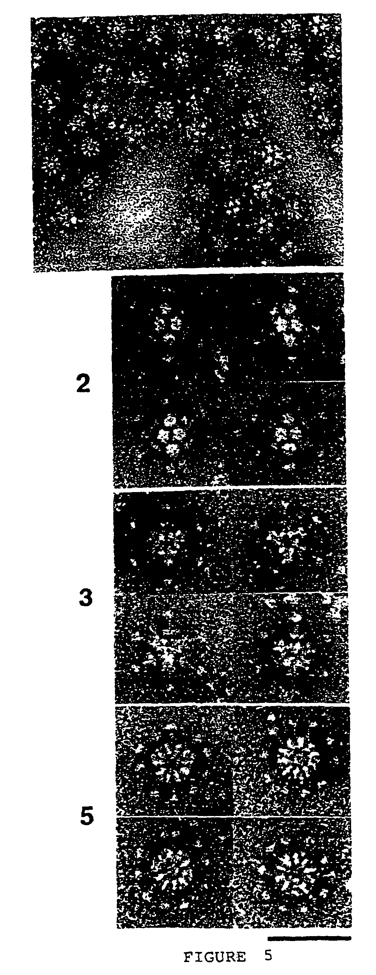

La figure 5 représente une micrographie électronique de dodécaèdre-fibre d'Ad3 coloré négativement ; la figure supérieure illustre un champ contenant un certain nombre de dodécaèdres dans des orientations différentes, alors que les figures numérotées 2, 3 et 5 illustrent des dodécaèdres sur un film-support selon leurs différents axes de symétrie (d'ordre 2, d'ordre 3 et d'ordre 5, respectivement) ; dans la figure illustrant l'axe de symétrie d'ordre 5, 10 fibres sont visibles, alors que les 11ème et 12ème fibres sont probablement dans le plan vertical par rapport au plan du papier.

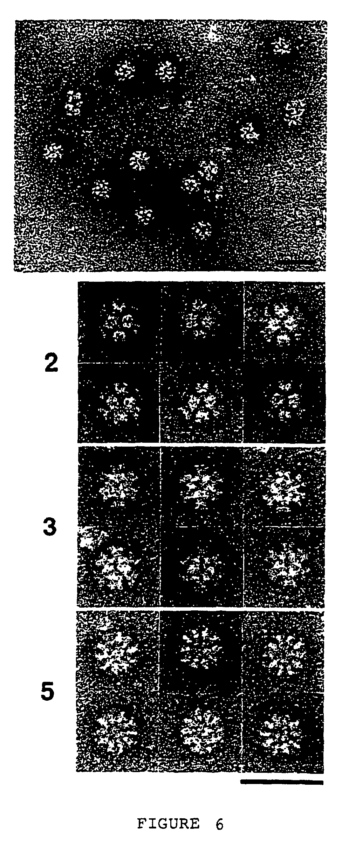

La figure 6 représente une micrographie électronique de dodécaèdre-base d'Ad3 coloré négativement ; la figure supérieure illustre un champ contenant un certain nombre de dodécaèdres dans des orientations différentes, alors que les figures numérotées 2, 3 et 5 illustrent des dodécaèdres sur un film-support selon leurs différents axes de symétrie (d'ordre 2, d'ordre 3 et d'ordre 5, respectivement).

Ces fractions contiennent des particules sphériques consistant en pentons (figure 5), associés en dodécaèdre, par l'intermédiaire de leurs bases.

Dans les figures 5 et 6, les différents axes de symétrie d'ordre 2, d'ordre 3 et d'ordre 5, sont aisément repérables.

Le diamètre du dodécaèdre avec les fibres (entre les têtes opposées de 2 fibres) est de 49 nm, alors que le diamètre de la partie base (de la partie supérieure d'une base à la partie supérieure de la base opposée) est de 27,8 nm (diamètre presque identique au diamètre du dodécaèdre sans fibre (27,5 nm)). Les pentons des dodécaèdres dissociés que l'on peut trouver, présentent une taille de 21,4 ± 1 nm (n=24), mesurée de la partie inférieure de la base à l'extrémité de la tête de fibre.

L'intérieur du dodécaèdre est constitué d'une cavité ayant un volume interne d'environ 350 nm³.

### 7. Rôle de la base du penton dans la formation du dodécaèdre

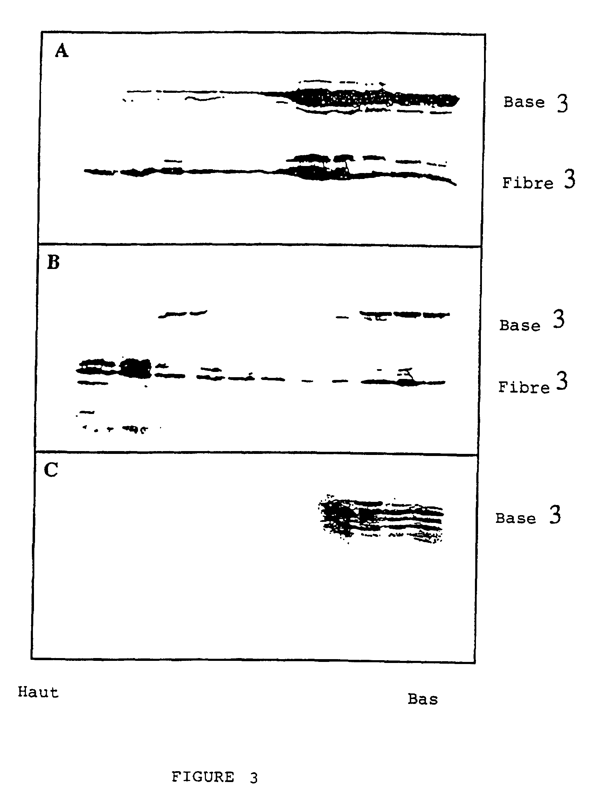

Les dodécaèdres formés à partir de bases du penton et de fibres peuvent être formés dans des cellules d'insecte, non seulement à travers le processus de co-expression à partir d'un baculovirus portant deux gènes, mais également à partir d'un co-infection avec deux baculovirus, chacun de ceux-ci portant un gène (figures 3A, B).

C'est cette approche qui a été choisie pour étudier le rôle de la base du penton dans la formation du dodécaèdre.

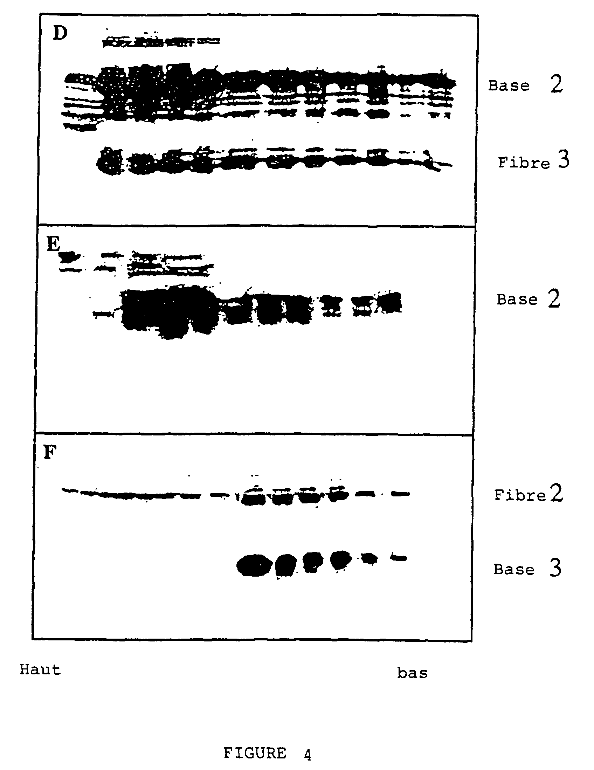

Lorsqu'une base Ad2 est utilisée avec une fibre Ad2 ou une fibre Ad3, les protéines obtenues dans les fractions de saccharose de densité élevée, contiennent des agrégats non-structurés de pentons (figure 4D) ou de bases (figure 4E).

Des pentons d'adénovirus chimériques peuvent être formés *in vitro,* par incubations de fibres purifiées et de bases du penton dérivées de différents sérotypes.

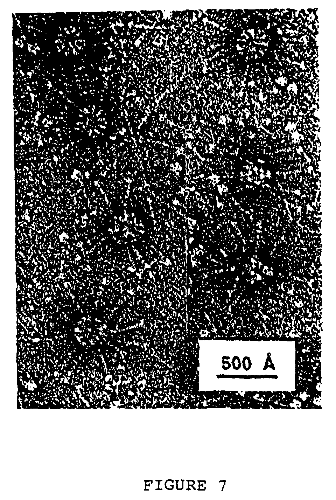

A partir d'une telle méthode, on a obtenu des dodécaèdres consistant uniquement en bases. Après incubation d'un dodécaèdre fait uniquement de bases Ad3 avec des fibres Ad2 natives, isolées de cellules infectés par Ad2, on obtient des dodécaèdres portant les fibres hétérologues insérées (figures 4 et 7).

Ces expériences montrent qu'en ce qui concerne l'étude des adénovirus Ad2 et Ad3, la formation d'un dodécaèdre dépend de la présence de la base Ad3 et non de la base Ad2.

Lorsque la base du penton Ad3 est exprimée seule dans le système baculovirus, on la retrouve pratiquement exclusivement sous la forme dodécaèdrique (Figure 3C).

La formation de dodécaèdre chimérique, lorsque des fibres Ad2 sont insérées dans un dodécaèdre-base préformé, confirme les résultats précédents concernant la formation de pentons chimériques.

Les figures 3 et 4 illustrent le rôle de la base du penton dans la formation du dodécaèdre. L'analyse en *Western blot* des différentes fractions du gradient de densité du saccharose est réalisée comme illustré à l'exemple 1 : figure 3A : co-expression de la base et de la fibre Ad3, à partir d'un baculovirus ; figure 3B : co-expression de la base et de la fibre Ad3, à partir d'une coinfection avec deux baculovirus, chacun portant l'un des gènes ; figure 3C : expression de la base Ad3 ; figure 4D : expression de la base Ad2 et de la fibre Ad3, à partir d'une coinfection avec deux baculovirus portant chacun un gène ; figure 4E : expression de la base Ad2 ; figure 4F : formation *in vitro* d'un dodécaèdre par incubation d'un dodécaèdre-base (Ad3), préformé *in vivo,* avec des fibres Ad2 natives.

### EXEMPLE 2 : Accumulation intracellulaire de dodécaèdres.

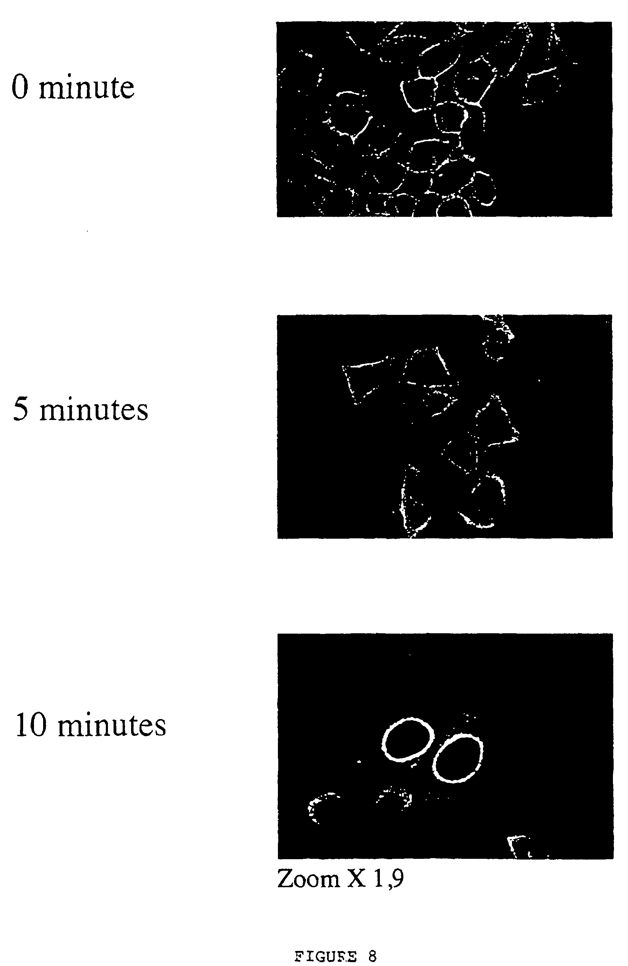

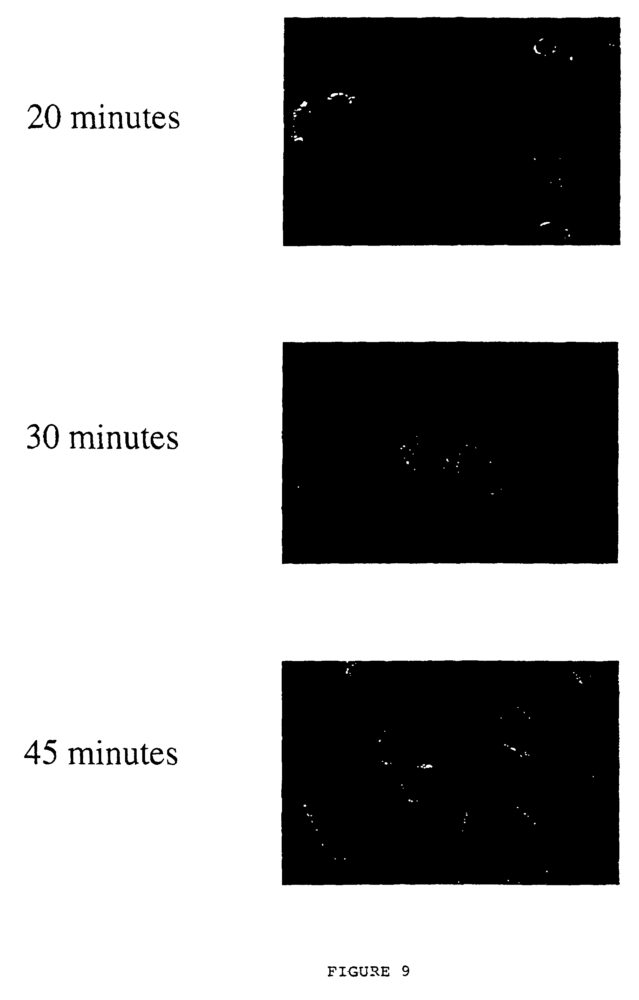

Les figures 8 et 9 sont des photos qui illustrent l'internalisation du dodécaèdre dans les cellules HeLa.

Pour réaliser ces photos, des portions de 5.10⁴ cellules HeLa sont cultivées dans un milieu DMEM contenant 10 % de sérum de veau fétal, sur lamelles (diamètre : 1,2 cm). Chaque lamelle est incubée 2 heures à 4°C avec 1 µg de dodécaèdre dans 50 µl de PBS-BSA à 3 %. Les cellules sont lavées 2 fois avec du PBS froid et incubées à 37°C dans du PBS-BSA à 3 % pendant 0 à 45 min. Les cellules sont fixées et perméabilisées par un traitement au méthanol pendant 5 min à -20°C. Les lamelles sont ensuite incubées pendant 1 heure à 37°C avec un anticorps contre la fibre (1:200, 50 µl dans du PBS-BSA à 3 %/lamelle), lavées 2 fois avec du PBS, puis incubées pendant 30 min avec un anticorps conjugué à la fluorescéine (1:250, 50 µl dans du PBS-BSA à 3 %/lamelle). Après un dernier lavage dans du PBS, les lamelles sont montées sur une plaque de microscope avec une goutte de 1,4-diaza-bicyclo[2.2.2.]octane (Sigma) à 50 mg/ml dans du glycérol à 50 %-PBS à 50 %. Les observations sont réalisées sur un microscope confocal MRC600 (Bio-Rad).

Les figures 8 et 9 montrent qu'après incubation à 4°C, il y a seulement attachement du complexe protéique dodécaèdrique auxdites cellules, alors qu'à 37°C, il y a internalisation dudit complexe.

A 4°C, le dodécaèdre est à la surface des cellules (visible après coloration avec un anticorps anti-fibre) ; lorsque les cellules HeLa sont transférées à 37°C, les figures 8 et 9 illustrent l'évolution de l'intemalisation du dodécaèdre, en fonction du temps : jusqu'à 5 minutes, l'image est très similaire (pas d'internalisation) ; après 10-15 minutes (figure 8) d'incubation, on observe un transfert massif du dodécaèdre au voisinage de la membrane nucléaire. Une incubation plus longue (20-45 minutes, figure 9) montre un signal plus diffus, ce qui illustre l'évacuation cytoplasmique du dodécaèdre. Des résultats similaires sont obtenus lorsque le devenir du dodécaèdre est suivi avec un sérum anti-base.

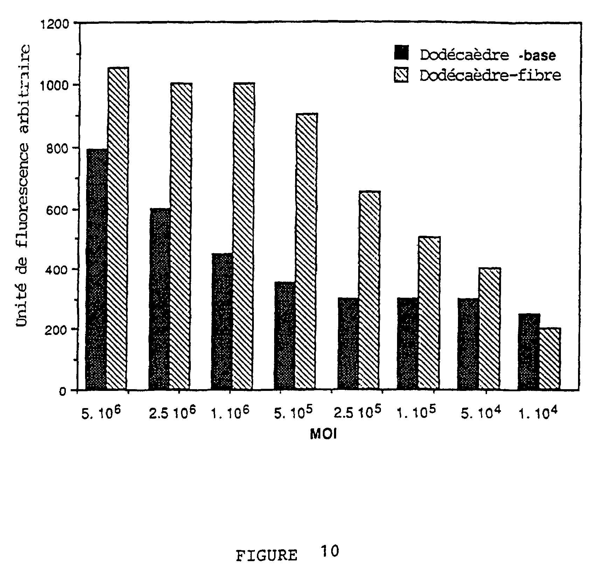

Une expérience similaire est réalisée avec un dodécaèdre constitué uniquement de bases d'Ad3. Aucun attachement ou internalisation n'est observé par microscopie électronique confocale lorsque l'on utilise un rapport molaire comparable à celui utilisé dans l'expérience précédente. Cependant, lorsque des quantités plus importantes de dodécaèdre-base sont utilisées, une internalisation est observée. Des quantités de dodécaèdre-base 10 à 20 fois plus importantes sont nécessaires pour obtenir un degré d'internalisation équivalent à celui obtenu avec le dodécaèdre-fibre. La figure 10 illustre les résultats obtenus après une heure d'intemalisation. Des résultats similaires sont obtenus après 30 minutes d'intemalisation.

Ces expériences montrent que l'absence de fibre peut être compensée par des quantités plus importantes de dodécaèdre-base ; cependant, la présence de fibre confère au dodécaèdre une efficacité supérieure, au moment de l'entrée des cellules.

Ces résultats peuvent être expliqués dans la mesure où pour l'adénovirus de sérotype 2 (Ad2), l'affinité de liaison de la fibre est 30 fois supérieure à celle de la base du penton (K_{d}s de 1,7 et 55 nM, respectivement).

A la température à laquelle se réalise l'attachement et l'internalisation du virus, on a montré que le dodécaèdre Ad3 s'attache aux cellules HeLa et que 10 à 20 minutes, à la température adaptée à l'entrée du virus, le dodécaèdre est retrouvé dans le cytoplasme, ciblé à la surface cytoplasmique de la membrane nucléaire. La cinétique globale semble être similaire à celle des premières étapes d'une infection virale, lorsque 20 minutes après l'attachement, environ 50 % de l'inoculum viral entrant (Ad5) est à la périphérie du noyau.

Dans le cas de l'entrée cellulaire du dodécaèdre Ad3, l'intégrité du dodécaèdre et de ses composants n'est pas compromise ni pendant les premières 20 minutes, ni plus tard (figures 8 et 9).

### EXEMPLE 3 : Transfection de cellules humaines avec le gène de la luciférase en utilisant le dodécaèdre Ad3 avec ou sans fibre et un peptide bifonctionnel : comparaison de l'efficacité de transfection d'un complexe protéique adénoviral selon l'invention, par rapport à un adénovirus recombinant ou des liposomes (DOTAP).

### - Préparation du peptide

Le peptide bifonctionnel synthétisé comporte 20 acides aminés correspondant à la partie N-terminale de la fibre Ad3 et 20 lysines du côté C-terminal, qui donneront un polycation pouvant fixer des polyanions comme l'ADN. séquence du polypeptide I : AKRARLSTSFNPVYPYEDES(K)₂₀ (SEQ ID N°5).

D'autres peptides bifonctionnels peuvent être construits : ils comportent, par exemple, l'extrémité N-terminale de n'importe quelle fibre d'adénovirus et se prolongent vers leur extrémité C-terminale soit par (1) une polyarginine ou (2) une partie ou une séquence complète de protéine de coeur de n'importe quel adénovirus, telle que protéine VII, protéine µ des adénovirus humains, ou des protéines de coeur homologues rencontrées dans les adénovirus d'autres animaux.

Les 4 peptides suivants ont également été synthétisés :
- polypeptide II (50 aminoacides) :
- polypeptide III (42 aminoacides) :
- polypeptide IV :
- polypeptide V :

Dans les polypeptides II et III, le fragment C-terminal, en gras, de 30 aminoacides, correspond à la séquence complète de la protéine VII de l'adénovirus aviaire sérotype 1 (FAV1 ou CELO) et la partie soulignée correspond à un fragment plus ou moins long de la fibre d'Ad3.

Dans les polypeptides IV et V, la partie C-terminale, en gras, correspond respectivement à la protéine µ de l'adénovirus humain et à la protéine µ de l'Ad1 aviaire CELO.

Tous ces peptides bifonctionnels peuvent être utilisés pour la transfection d'ADN, séparément ou en mélange.

### - Préparation du plasmide contenant l'ADN à transporter (substance active

Le plasmide codant pour le gène reporteur de la luciférase (pGL3-control vector) (Promega) est produit chez *E. coli* JM109 et purifié par Qiagen.

### - Méthode

Mélange 1 : Des échantillons de dodécaèdre avec ou sans fibre sont incubés dans un milieu DMEM avec 5 µg de peptide bifonctionnel pendant 15 minutes, à température ambiante, puis 1,5 µg de plasmide pGL3 portant le gène de la luciférase sont ajoutés.

Mélange 2 : par ailleurs, un mélange de DOTAP (Boehringer) et de 1,5 µg de plasmide pGL3, est préparé, comme précisé par le fournisseur, avec un rapport DOTAP/ADN de 4.

Des portions de 10⁵ cellules HeLa/puits, dans une plaque comprenant 24 puits, sont transfectées en parallèle, avec les mélanges ci-dessus et avec l'adénovirus recombinant Ad5luc, pendant une heure à 37°C.

L'émission de lumière est mesurée dans des lysats cellulaires, 48 heures après, à l'aide du kit Promega.

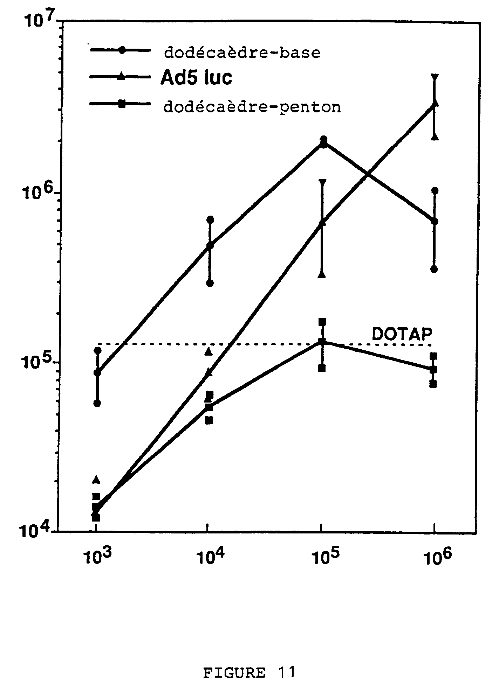

La figure 11 illustre les résultats obtenus.

### EXEMPLE 4 : Mise en évidence de l'interaction du peptide bifonctionnel avec le complexe protéinique adénoviral selon l'invention et l'ADN plasmidique à transporter.

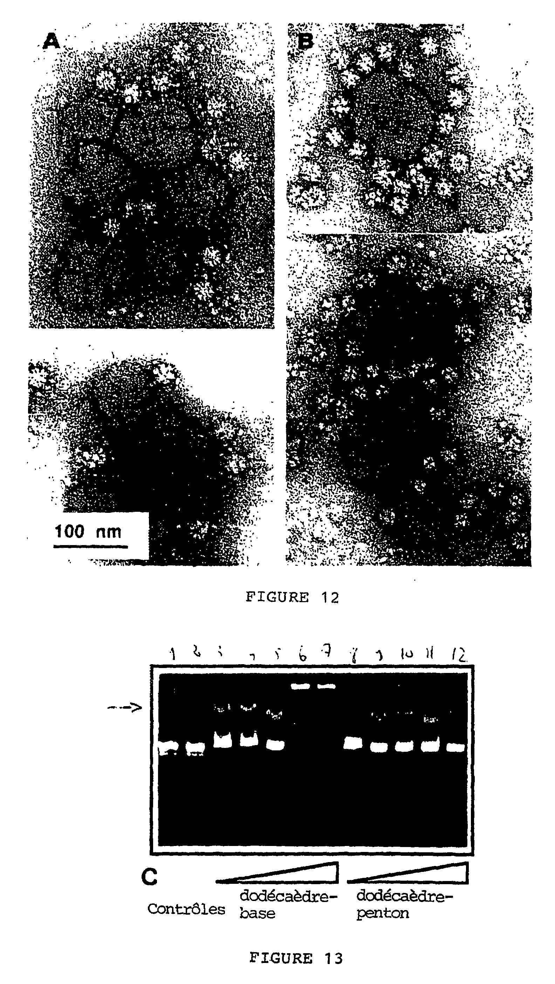

Des complexes ADN/peptide/dodécaèdre-penton et ADN/peptide/dodécaèdre-base sont préparés comme précisé dans les essais de transfection (voir exemple 3), sans ajout de milieu ; les micrographies électroniques de ces complexes sont illustrées aux figures 12A et 12B et montrent l'ADN dans une forme compacte avec le dodécaèdre-penton (A) le dodécaèdre-base (B). En parallèle, des quantités croissantes de dodécaèdre-base sont incubées avec 200 ng de peptide pendant 15 minutes à température ambiante ; 250 ng d'ADN plasmidique sont ajoutés, puis 5 minutes plus tard, les échantillons sont déposés sur un gel d'agarose à 1 % fait dans un tampon TBE, puis sont soumis à une électrophorèse pendant 1 heure à 50 volts.

L'ADN est révélé par coloration au bromure d'éthidium, suivi par une visualisation aux UV. Tous les échantillons contiennent de l'ADN plasmidique. La piste 1 correspond à de l'ADN plasmidique seul ; la piste 2 correspond à de l'ADN plasmidique mélangé au dodécaèdre-base (pas de peptide) ; les pistes 3 à 7 comprennent de l'ADN plasmidique, le peptide bifonctionnel et respectivement 1, 10, 100, 500 et 1 000 ng de dodécaèdre-base ; les pistes 8 à 12 comprennent de l'ADN plasmidique, le peptide bifonctionnel et respectivement 1, 10, 100, 500 et 1 000 ng de dodécaèdre-penton.

Cette figure montre bien que le peptide s'attache au dodécaèdre et au plasmide et modifie la structure : l'ADN plasmidique devient compact et la migration est retardée : présence d'une bande intermédiaire (voir flèche) ou même absence de migration (voir pistes 6 et 7).

## Revendications

1. Complexe protéique adénoviral, **caractérisé en ce qu'**il est constitué :
- soit de 12 pentons, comprenant chacun une fibre et une base de penton, à l'exclusion de tout autre élément constitutif du génome d'un adénovirus, lesquelles fibre(s) et base du penton sont dérivées soit du même adénovirus, soit d'adénovirus différents, lesdits pentons étant liés par les bases de penton et formant une structure en dodécaèdre, stable aux enzymes protéolytiques, lequel complexe présente un poids moléculaire compris entre 4,8.10⁶ et 6,6.10⁶ ;
- soit de 12 bases de penton, à l'exclusion de tout autre élément constitutif du génome d'un adénovirus, lesquelles bases de penton sont dérivées soit du même adénovirus, soit d'adénovirus différents, et forment une structure en dodécaèdre, stable aux enzymes protéolytiques et **en ce qu'**il présente un poids moléculaire compris entre 3,2.10⁶ et 4.10⁶.

2. Complexe protéique adénoviral selon la revendication 1, **caractérisé en ce que** lesdits adénovirus sont sélectionnés parmi les adénovirus humains et notamment l'adénovirus de type 2 (Ad2), l'adénovirus de type 3 (Ad3), l'adénovirus de type 5 (Ad5), l'adénovirus de type 4 (Ad4), l'adénovirus de type 7 (Ad7), l'adénovirus de type 9 (Ad9), l'adénovirus de type 11 (Ad11), l'adénovirus de type 15 (Ad15) ou les adénovirus entériques (Ad40 et Ad41) et les adénovirus aviaires.

3. Complexe protéique adénoviral selon la revendication 1 ou la revendication 2, **caractérisé en ce que** au moins l'un de ses constituants (base et/ou fibre) peut être modifié pour augmenter l'affinité vis-à-vis d'un type cellulaire particulier.

4. Complexe protéique adénoviral selon l'une quelconque des revendications 1 à 3, **caractérisé en ce qu'**il comprend en outre un hexon ou une autre protéine virale.

5. Composition pharmaceutique, **caractérisée en ce qu'**elle comprend un complexe protéique adénoviral selon l'une quelconque des revendications 1 à 4 et une autre substance chimique.

6. Composition selon la revendication 5, **caractérisée en ce que** ladite substance chimique est sélectionnée dans le groupe constitué par des séquences nucléiques, des protéines, des peptides et des substances chimiques pharmacologiquement actives.

7. Composition selon la revendication 5 ou la revendication 6, **caractérisée en ce que** lorsque la substance chimique est une protéine, elle présente, de préférence, des propriétés immunoprotectrices et antigéniques.

8. Composition selon la revendication 5 ou la revendication 6, **caractérisée en ce que** lorsque la substance chimique est une séquence nucléique, elle est sélectionnée parmi les gènes qui codent pour un polypeptide présentant une activité thérapeutique, les séquences anti-sens et les ribozymes.

9. Composition selon la revendication 8, **caractérisée en ce que** lorsqu'elle comprend une séquence codante, elle comprend en outre un promoteur actif pour l'expression du polypeptide, lequel promoteur est sélectionné dans le groupe constitué par des promoteurs constitutifs et des promoteurs inductibles.

10. Composition selon l'une quelconque des revendications 5, 6, 8 ou 9, **caractérisée en ce que** lorsque ladite substance chimique est une séquence d'acide nucléique ou une protéine, elle peut être associée à un ligand sélectionné parmi les peptides dont la partie N-terminale comprend la séquence en aminoacides N-terminale d'une fibre d'adénovirus de n'importe quel sérotype et dont la partie C-terminale comprend notamment une polylysine, une polyarginine ou une partie ou la séquence complète d'une protéine de coeur de n'importe quel adénovirus, une cystéine ou un complexe transferrine/poly-L-lysine.

11. Composition selon l'une quelconque des revendications 5 à 10, **caractérisée en ce qu'**elle comprend, en outre, un véhicule pharmaceutiquement acceptable.

12. Composition comprenant un complexe protéique adénoviral selon l'une quelconque des revendications 1 à 4 et une substance chimique sélectionnée parmi les séquences nucléotidiques, les protéines et les substances chimiques actives pharmacologiquement, selon l'une quelconque des revendications 5 à 11, en tant que médicament.

13. Composition comprenant un complexe protéique adénoviral selon l'une quelconque des revendications 1 à 4 et une substance chimique sélectionnée parmi les protéines selon la revendication 6 ou la revendication 7, en tant que préparation vaccinale.

14. Composition selon l'une quelconque des revendications 5 à 11, comprenant un complexe protéique dodécaèdrique adénoviral et une substance chimique sélectionnée parmi les séquences nucléotidiques, les substances immunogènes, et les substances chimiques pharmacologiquement actives sélectionnées dans le groupe constitué par les substances anticancéreuses, pour son utilisation dans le traitement des maladies humaines ou animales impliquant des cellules exprimant des récepteurs spécifiques des fibres d'adénovirus et/ou des cellules exprimant les récepteurs des intégrines, notamment des intégrines αᵥβ₃ ou αᵥβ₅, telles que les cellules épithéliales, les cellules endothéliales, les plaquettes sanguines, les cellules lymphoïdes et les cellules cancéreuses et libérant une quantité efficace de ladite substance chimique active aux dites cellules.

15. Procédé de préparation d'un complexe protéique dodécaédrique adénoviral selon l'une quelconque des revendications 1 à 4, **caractérisé en ce qu'**il comprend les étapes suivantes :
(1) le clonage séparé ou simultané du (ou des) gène(s) codant pour la/les fibre(s) d'un adénovirus et du gène codant pour la base du penton d'un adénovirus, lesdits gènes étant issus soit du même adénovirus, soit d'adénovirus différents, dans au moins un vecteur baculovirus, pour l'obtention de plusieurs plasmides recombinants contenant soit un gène, soit deux gènes ou pour l'obtention d'un plasmide recombinant contenant simultanément deux ou trois gènes ;
(2) la co-transfection du ou des plasmides recombinants et d'un fragment linéarisé d'ADN de baculovirus, dans une cellule d'insecte ;
(3) le criblage et la sélection des clones recombinants de baculovirus exprimant, séparément ou à la fois la/les fibres et la base du penton ;
(4) la purification des clones sélectionnés correspondant à des baculovirus recombinants replicables, contenant un ou plusieurs desdits gènes, issus d'au moins un adénovirus et exprimant les protéines correspondantes ;
(5) l'extraction des protéines exprimées par lesdits baculovirus recombinants ; et
(6) la purification du complexe protéique dodécaèdrique, par application de l'extrait obtenu en (5) sur un gradient de saccharose, dont la fourchette de concentrations varie en fonction du poids moléculaire et de la densité des dodécaèdres obtenus et récupération des fractions, dans la zone des concentrations élevées en saccharose.

16. Procédé selon la revendication 15, **caractérisé en ce que** le vecteur baculovirus utilisé comprend une cassette de double, triple ou multiple expression.

17. Procédé selon la revendication 15 ou la revendication 16, **caractérisé en ce que** les cellules d'insecte de l'étape (2) sont sélectionnées dans le groupe constitué par les cellules de *Spodoptera frugiperda* et les cellules de *Trichoplusia ni.*

18. Procédé selon l'une quelconque des revendications 15 à 17, **caractérisé en ce que** la co-transfection de l'étape (2) est mise en oeuvre dans des cellules de *Spodoptera frugiperda* et le procédé comprend une deuxième étape (4') de transfection des clones purifiés de baculovirus recombinants obtenus à l'étape (4), dans des cellules d'insecte *Trichoplusia ni.*

19. Procédé selon la revendication 17 ou la revendication 18, **caractérisé en ce que** la cellule d'insecte *Spodoptera frugiperda* de l'étape (2) est sélectionnée dans le groupe constitué par les cellules Sf21 et les cellules Sf9.

20. Procédé selon la revendication 18, **caractérisé en ce que** la transfection de l'étape (4') comprend la co-transfection de baculovirus recombinants qui contiennent les deux gènes issus d'adénovirus précités, éventuellement associés à des baculovirus recombinants qui contiennent un seul des deux gènes issus d'adénovirus.

21. Procédé de préparation d'un complexe protéique dodécaédrique adénoviral, dérivé d'un adénovirus selon l'une quelconque des revendications 1 à 4, **caractérisé en ce qu'**il comprend :
- une étape (1) selon la revendication 15,
- une étape (2) d'isolement des plasmides recombinants et de transfection de cellules d'insecte avec lesdits plasmides recombinants, et
- des étapes (3) à (6) selon la revendication 15.

## Patentansprüche

1. Adenoviraler Proteinkomplex, **dadurch gekennzeichnet, dass** er gebildet ist:
- entweder von 12 Pentonen, die jeweils eine Pentonfaser und eine Pentonbasis aufweisen, unter Ausschluss jedes anderen wesentlichen Bestandteils des Genoms eines Adenovirus, wobei die Faser(n) und die Basis des Pentons entweder von demselben Adenovirus oder von unterschiedlichen Adenoviren stammen, wobei die Pentone über die Pentonbasen verbunden sind und eine gegenüber proteolytischen Enzymen beständige Dodekaederstruktur bilden, wobei der genannte Komplex ein Molekulargewicht zwischen 4,8.10⁶ und 6,6.10⁶ aufweist;
- oder von 12 Pentonbasen, unter Ausschluss jedes anderen wesentlichen Bestandteils des Genoms eines Andenovirus, wobei die Pentonbasen entweder von demselben Adenovirus oder von unterschiedlichen Adenoviren stammen und eine gegenüber proteolytischen Enzymen beständige Dodekaederstruktur bilden, und dass er ein Molekulargewicht zwischen 3,2.10⁶ und 4.10⁶ aufweist.

2. Adenoviraler Proteinkomplex nach Anspruch 1, **dadurch gekennzeichnet, dass** die Adenoviren ausgewählt sind aus den menschlichen Adenoviren und insbesondere dem Adenovirus Typ 2 (Ad2), Adenovirus Typ 3 (Ad3), Adenovirus Typ 5 (Ad5), Adenovirus Typ 4 (Ad4), Adenovirus Typ 7 (Ad7), Adenovirus Typ 9 (Ad9), Adenovirus Typ 11 (Ad11), Adenovirus Typ 15 (Ad15) oder den Enteroadenoviren (Ad40 und Ad41) und den aviären Adenoviren.

3. Adenoviraler Proteinkomplex nach Anspruch 1 oder Anspruch 2, **dadurch gekennzeichnet, dass** wenigstens einer seiner Bestandteile (Basis und/oder Faser) verändert sein kann, um die Affinität gegenüber einem besonderen Zelltyp zu erhöhen.

4. Adenoviraler Proteinkomplex nach irgendeinem der Ansprüche 1 bis 3, **dadurch gekennzeichnet, dass** er außerdem ein Hexon oder ein anderes virales Protein aufweist.

5. Pharmazeutische Zusammensetzung, **dadurch gekennzeichnet, dass** sie einen adenoviralen Proteinkomplex nach irgendeinem der Ansprüche 1 bis 4 sowie eine weitere chemische Substanz enthält.

6. Zusammensetzung nach Anspruch 5, **dadurch gekennzeichnet, dass** die chemische Substanz ausgewählt ist aus der Gruppe bestehend aus Nukleinsequenzen, Proteinen, Peptiden und pharmakologisch aktiven chemischen Substanzen.

7. Zusammensetzung nach Anspruch 5 oder Anspruch 6, **dadurch gekennzeichnet, dass** dann, wenn die chemische Substanz ein Protein ist, es vorzugsweise immunoprotektive und antigene Eigenschaften aufweist.

8. Zusammensetzung nach Anspruch 5 oder Anspruch 6, **dadurch gekennzeichnet, dass** dann, wenn die chemische Substanz eine Nukleinsequenz ist, sie ausgewählt ist aus Genen, die für ein Polypeptid mit therapeutischer Aktivität codieren, Antisense-Sequenzen und Ribozymen.

9. Zusammensetzung nach Anspruch 8, **dadurch gekennzeichnet, dass** wenn sie eine codierende Sequenz enthält, sie außerdem einen aktiven Promotor für die Expression des Polypeptids aufweist, wobei der Promotor ausgewählt ist aus der Gruppe bestehend aus konstitutiven Promotoren und induzierbaren Promotoren.

10. Zusammensetzung nach irgendeinem der Ansprüche 5, 6, 8 oder 9, **dadurch gekennzeichnet, dass** wenn die chemische Substanz eine Nukleinsäuresequenz oder ein Protein ist, sie mit einem Liganden assoziiert sein kann, welcher ausgewählt ist aus den Peptiden, deren N-terminaler Teil die N-terminale Aminosäure-Sequenz einer Adenovirus-Faser irgendeines Serotyps enthält und deren C-terminaler Teil insbesondere ein Polylysin, ein Polyarginin oder einen Teil oder die vollständige Sequenz eines Kemproteins irgendeines Adenovirus, ein Cystein oder einen Transferrin/Poly-L-Lysin-Komplex enthält.

11. Zusammensetzung nach irgendeinem der Ansprüche 5 bis 10, **dadurch gekennzeichnet, dass** sie weiterhin ein pharmazeutisch akzeptables Vehikel enthält.

12. Zusammensetzung mit einem adenoviralen Proteinkomplex nach irgendeinem der Ansprüche 1 bis 4 und einer chemischen Substanz, welche ausgewählt ist aus den Nukleotidsequenzen, Proteinen und pharmakologisch aktiven chemischen Substanzen, nach irgendeinem der Ansprüche 5 bis 11, als Medikament.

13. Zusammensetzung mit einem adenoviralen Proteinkomplex nach irgendeinem der Ansprüche 1 bis 4 und einer chemischen Substanz, welche ausgewählt ist aus den Proteinen nach Anspruch 6 oder Anspruch 7, als Impfpräparat.

14. Zusammensetzung nach irgendeinem der Ansprüche 5 bis 11, die einen adenoviralen dodekaedrischen Proteinkomplex und eine chemische Substanz ausgewählt aus Nukleotidsequenzen, immunogenen Substanzen und pharmakologisch aktiven chemischen Substanzen ausgewählt aus der Gruppe bestehend aus Antikrebs-Substanzen enthält, für ihre Verwendung bei der Behandlung menschlicher oder tierischer Krankheiten, die Zellen implizieren, welche spezifische Rezeptoren der Adenovirus-Fasem exprimieren und/oder Zellen, welche die Rezeptoren der Integrine, insbesondere der Integrine αᵥβ₃ oder αᵥβ₅ exprimieren, wie Epithelzellen, Endothelzellen, Blutplättchen, lymphoide Zellen und Krebszellen, und die eine wirksame Menge der aktiven chemischen Substanz an die genannten Zellen freisetzt.

15. Verfahren zur Zubereitung eines adenoviralen dodekaedrischen Proteinkomplexes nach irgendeinem der Ansprüche 1 bis 4, **dadurch gekennzeichnet**, das es die folgenden Schritte umfasst:
(1) getrennte oder gleichzeitige Klonierung des (oder der) Gens (Gene), das bzw. die für die Faser(n) eines Adenovirus codiert bzw. codieren, und des Gens, welches für die Basis des Pentons eines Adenovirus codiert, wobei die Gene entweder von demselben Adenovirus oder von unterschiedlichen Adenoviren stammen, in wenigstens einen Baculovirus-Vektor, für den Erhalt mehrerer rekombinanter Plasmide, die entweder ein Gen oder zwei Gene enthalten, oder für den Erhalt eines rekombinanten Plasmids, welches gleichzeitig zwei oder drei Gene enthält;
(2) Kotransfektion des oder der rekombinanten Plasmids bzw. Plasmide und eines linearisierten Baculovirus-DNA-Fragments in einer Insektenzelle;
(3) Klassieren und Auswählen der rekombinanten Baculovirus-Klone, die getrennt oder gleichzeitig die Faser(n) und die Basis des Pentons exprimieren;
(4) Reinigen der ausgewählten Klone, die rekombinanten, replizierbaren Baculoviren entsprechen, die ein oder mehrere der von wenigstens einem Adenovirus stammenden Gene enthalten und die die entsprechenden Proteine exprimieren;
(5) Extraktion der durch die rekombinanten Baculoviren exprimierten Proteine; und
(6) Reinigen des dodekaedrischen Proteinkomplexes durch Anwendung des in (5) erhaltenen Extrakts an einem Saccharosegradienten, dessen Konzentrationsbereich in Abhängigkeit von dem Molekulargewicht und der Dichte der erhaltenen Dodekaeder variiert, und Rückgewinnung der Fraktionen im Bereich der erhöhten Saccharosekonzentrationen.

16. Verfahren nach Anspruch 15, **dadurch gekennzeichnet, dass** der verwendete Baculovirus-Vektor eine Kassette zur zweifachen, dreifachen oder mehrfachen Expression enthält.

17. Verfahren nach Anspruch 15 oder Anspruch 16, **dadurch gekennzeichnet, dass** die Insektenzellen des Schrittes (2) ausgewählt sind aus der Gruppe bestehend aus *Spodoptera frugiperda*-Zellen und *Trichoplusia ni*-Zellen.

18. Verfahren nach irgendeinem der Ansprüche 15 bis 17, **dadurch gekennzeichnet, dass** die Kotransfektion des Schrittes (2) in *Spodoptera frugiperda*-Zellen vollzogen wird und dass das Verfahren einen zweiten Schritt (4') zur Transfektion der in Schritt (4) erhaltenen gereinigten Klone von rekombinanten Baculoviren in *Trichoplusia ni*-Insektenzellen umfasst.

19. Verfahren nach Anspruch 17 oder Anspruch 18, **dadurch gekennzeichnet, dass** die *Spodoptera frugiperda*-Insektenzelle des Schrittes (2) ausgewählt ist aus der Gruppe bestehend aus Sf21-Zellen und Sf9-Zellen.

20. Verfahren nach Anspruch 18, **dadurch gekennzeichnet, dass** die Transfektion des Schrittes (4') die Kotransfektion von rekombinanten Baculoviren umfasst, welche die aus vorgenannten Adenoviren stammenden zwei Gene enthalten und eventuell mit rekombinanten Baculoviren assoziiert sind, welche ein einziges der beiden aus Adenoviren stammenden Gene enthalten.

21. Verfahren zur Zubereitung eines adenoviralen dodekaedrischen Proteinkomplexes, stammend aus einem Adenovirus nach irgendeinem der Ansprüche 1 bis 4, **dadurch gekennzeichnet, dass** es umfasst:
- einen Schritt (1) gemäß Patentanspruch 15,
- einen Schritt (2) zur Isolierurig der rekombinanten Plasmide und zur Transfektion von Insektenzellen mit den rekombinanten Plasmiden, und
- Schritte (3) bis (6) gemäß Patentanspruch 15.

## Claims

1. Adenoviral protein complex, **characterized in that** it is formed:
- either of 12 pentons, each comprising one fibre and one penton base, with the exclusion of any other constitutive element of the genome of an adenovirus, which fibre(s) and penton base are derived either from the same adenovirus, or from different adenoviruses, the said pentons being bound by the penton bases and forming a dodecahedral structure, stable to proteolytic enzymes, which complex has a molecular weight of between 4.8 × 10⁶ and 6.6 × 10⁶;
- or of 12 penton bases, with the exclusion of any other constitutive element of an adenovirus, which penton bases are derived either from the same adenovirus, or from different adenoviruses, and form a dodecahedral structure, stable to proteolytic enzymes and **in that** it has a molecular weight of between 3.2 × 10⁶ and 4 × 10⁶.

2. Adenoviral protein complex according to Claim 1, **characterized in that** the said adenoviruses are selected from human adenoviruses and especially type 2 adenovirus (Ad2), type 3 adenovirus (Ad3), type 5 adenovirus (Ad5), type 4 adenovirus (Ad4), type 7 adenovirus (Ad7), type 9 adenovirus (Ad9), type 11 adenovirus (Ad11), type 15 adenovirus (Ad15) or the enteric adenoviruses (Ad40 and Ad41) and the avian adenoviruses.

3. Adenoviral protein complex according to Claim 1 or Claim 2, **characterized in that** at least one of its constituents (base and/or fibre) can be modified to increase the affinity with respect to a particular cell type.

4. Adenoviral protein complex according to any one of Claims 1 to 3, **characterized in that** it additionally comprises one hexon or another viral protein.

5. Pharmaceutical composition, **characterized in that** it comprises an adenoviral protein complex according to any one of Claims 1 to 4 and one other chemical substance.

6. Composition according to Claim 5, **characterized in that** the said chemical substance is selected from the group formed by nucleic sequences, proteins, peptides and pharmacologically active chemical substances.

7. Composition according to Claim 5 or Claim 6, **characterized in that** when the chemical substance is a protein, it preferably has immunoprotective and antigenic properties.

8. Composition according to Claim 5 or Claim 6, **characterized in that** when the chemical substance is a nucleic sequence, it is selected from the genes which code for a polypeptide having a therapeutic activity, the anti-sense sequences and the ribozymes.

9. Composition according to Claim 8, **characterized in that** when it comprises a coding sequence, it additionally comprises an active promoter for the expression of the polypeptide, which promoter is selected from the group formed by constitutive promoters and inducible promoters.

10. Composition according to any one of Claims 5, 6, 8 and 9, **characterized in that** when the said chemical substance is a nucleic acid sequence or a protein, it can be combined with a ligand selected from the peptides whose N-terminal part comprises the N-terminal amino acid sequence of an adenovirus fibre of no matter which serotype and whose C-terminal part especially comprises a polylysine, a polyarginine or a part or the complete sequence of a core protein of any adenovirus, a cysteine or a transferrin/poly-L-lysine complex.

11. Composition according to any one of Claims 5 to 10, **characterized in that** it comprises, in addition, one pharmaceutically acceptable vehicle.

12. Composition comprising an adenoviral protein complex according to any one of Claims 1 to 4 and a chemical substance selected from the nucleotide sequences, the proteins and the pharmacologically active chemical substances, according to any one of Claims 5 to 11, as a medicament.

13. Composition comprising an adenoviral protein complex according to any one of Claims 1 to 4 and a chemical substance selected from the proteins according to Claim 6 or Claim 7, as a vaccine preparation.

14. Composition according to any one of Claims 5 to 11, comprising a dodecahedral adenoviral protein complex and a chemical substance selected from the nucleotide sequences, the immunogenic substances, and the pharmacologically active chemical substances selected from the group formed by the anticancer substances, for use thereof in the treatment of human or animal illnesses involving cells expressing specific receptors of adenovirus fibres and/or of cells expressing integrin receptors, especially αᵥβ₃ or αᵥβ₅ integrins, such as epithelial cells, endothelial cells, blood platelets, lymphoid cells and cancerous cells and liberating an efficacious quantity of the said active chemical substance to the said cells.

15. Process for the preparation of a dodecahedral adenoviral protein complex according to any one of Claims 1 to 4, **characterized in that** it comprises the following steps:
(1) the separate or simultaneous cloning of the gene(s) coding for the fibre(s) of an adenovirus and of the gene coding for the penton base of an adenovirus, the said genes being either from the same adenovirus, or from different adenoviruses, in at least one baculovirus vector, to obtain several recombinant plasmids containing either one gene, or two genes, or to obtain a recombinant plasmid simultaneously containing two or three genes;
(2) the co-transfection of the recombinant plasmid(s) and of a linearized baculovirus DNA fragment in an insect cell;
(3) the screening and the selection of recombinant baculovirus clones expressing, separately or at the same time, the fibre(s) and the penton base;
(4) the purification of the selected clones corresponding to replicable recombinant baculoviruses, containing one or more of the said genes, from at least one adenovirus and expressing the corresponding proteins;
(5) the extraction of the proteins expressed by the said recombinant baculoviruses; and
(6) the purification of the dodecahedral protein complex, by application of the extract obtained in (5) to a sucrose gradient whose range of concentrations varies as a function of the molecular weight and of the density of the dodecahedrons obtained and recovery of the fractions in the area of high sucrose concentrations.

16. Process according to Claim 15, **characterized in that** the baculovirus vector used comprises a double, triple or multiple expression cassette.

17. Process according to Claim 15 or Claim 16, **characterized in that** the insect cells of step (2) are selected from the group formed by the cells of *Spodoptera frugiperda* and the cells of *Trichoplusia ni.*

18. Process according to any one of Claims 15 to 17, **characterized in that** the co-transfection of step (2) is carried out in cells of *Spodoptera frugiperda* and the process comprises a second step (4') of transfection of the purified clones of recombinant baculoviruses obtained in step (4) in *Trichoplusia ni* insect cells.

19. Process according to Claim 17 or Claim 18, **characterized in that** the *Spodoptera frugiperda* insect cell of step (2) is selected from the group formed by the Sf21 cells and the Sf9 cells.

20. Process according to Claim 18, **characterized in that** the transfection of step (4') comprises the co-transfection of recombinant baculoviruses which contain the two genes from abovementioned adenoviruses, optionally combined with recombinant baculoviruses which contain only one of the two genes from adenoviruses.

21. Process for the preparation of a dodecahedral adenoviral protein complex derived from an adenovirus according to any one of Claims 1 to 4, **characterized in that** it comprises:
- a step (1) according to Claim 15,
- an isolation step (2) of the recombinant plasmids and of transfection of insect cells with the said recombinant plasmids, and
- steps (3) to (6) according to Claim 15.
